(19) Europäisches Patentamt
European Patent Office
Office européen des brevets

(11) EP 1 100 443 B1

(12) **EUROPÄISCHE PATENTSCHRIFT**

(45) Veröffentlichungstag und Bekanntmachung des Hinweises auf die Patenterteilung:
**02.11.2005 Patentblatt 2005/44**

(21) Anmeldenummer: **99940061.7**

(22) Anmeldetag: **27.07.1999**

(51) Int Cl.[7]: **A61K 7/06**

(86) Internationale Anmeldenummer:
**PCT/EP1999/005374**

(87) Internationale Veröffentlichungsnummer:
**WO 2000/007552 (17.02.2000 Gazette 2000/07)**

(54) **HAARBEHANDLUNGSMITTEL**

HAIR TREATMENT PREPARATION

PRODUIT CAPILLAIRE

(84) Benannte Vertragsstaaten:
**AT BE CH CY DE DK ES FI FR GB GR IE IT LI LU MC NL PT SE**

(30) Priorität: **05.08.1998 DE 19835327**

(43) Veröffentlichungstag der Anmeldung:
**23.05.2001 Patentblatt 2001/21**

(73) Patentinhaber: **Henkel Kommanditgesellschaft auf Aktien**
**40589 Düsseldorf (DE)**

(72) Erfinder:
- **HÖFFKES, Horst**
  **D-40595 Düsseldorf (DE)**
- **BERGMANN, Bettina**
  **D-47269 Duisburg (DE)**
- **NELLES, Karin**
  **D-40789 Monheim (DE)**

(56) Entgegenhaltungen:
**EP-A- 0 648 833**
**EP-A- 0 830 856**
**DE-A- 4 430 522**
**FR-A- 2 203 806**
**GB-A- 2 140 297**
**US-A- 3 891 385**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 100 443 B1

Printed by Jouve, 75001 PARIS (FR)

## Beschreibung

Die Erfindung betrifft eine spezielle Wirkstoffkombination zur Verbesserung der Kämmbarkeit keratinischer Fasern sowie spezielle Haarbehandlungsmittel, die diese Wirkstoffkombination enthalten.

Die Reinigung und Pflege der Haare ist ein wichtiger Bestandteil der menschlichen Körperpflege. Sowohl die Reinigung der Haare mit Shampoos als auch die dekorative Gestaltung der Frisur beispielsweise durch Färben oder Dauerwellen sind Eingriffe, die die natürliche Struktur und die Eigenschaften der Haare beeinflussen. So können anschließend an eine solche Behandlung beispielsweise die Naß- und Trockenkämmbarkeit des Haares, der Halt und die Fülle des Haares unbefriedigend sein oder die Haare einen erhöhten Spliß aufweisen.

Es ist daher seit langem üblich, die Haare einer speziellen Nachbehandlung zu unterziehen. Dabei werden, üblicherweise in Form einer Spülung, die Haare mit speziellen Wirkstoffen, beispielsweise quaternären Ammoniumsalzen oder speziellen Polymeren, behandelt. Durch diese Behandlung werden je nach Formulierung Kämmbarkeit, Halt und Fülle der Haare verbessert und die Splißrate verringert.

Weiterhin wurden in jüngster Zeit sogenannte Kombinationspräparate entwickelt, um den Aufwand der üblichen mehrstufigen Verfahren, insbesondere bei der direkten Anwendung durch Verbraucher, zu verringern.

Diese Präparate enthalten neben den üblichen Komponenten, beispielsweise zur Reinigung der Haare, zusätzlich Wirkstoffe, die früher den Haarnachbehandlungsmitteln vorbehalten waren. Der Konsument spart somit einen Anwendungsschritt; gleichzeitig wird der Verpackungsaufwand verringert, da ein Produkt weniger gebraucht wird.

Die zur Verfügung stehenden Wirkstoffe sowohl für separate Nachbehandlungsmittel als auch für Kombinationspräparate können noch nicht alle Wünsche der Verbraucher erfüllen.

So ist beispielsweise die biologische Abbaubarkeit der quaternären Ammoniumverbindungen unbefriedigend und ihre häufig mangelnde Kompatibilität mit Aniontensiden schränkt die Formulierungsmöglichkeiten stark ein. Außerdem sind quaternäre Tenside in einzelnen Fällen haut- und schleimhautreizend. Die gleichen Nachteile gelten auch für die kationischen Polymeren. Weiterhin können bei diesen Polymeren unerwünschte Kumulationen auf dem Haar auftreten.

Alkylphosphonate und Alkylphosphate sind zwar mit anionischen Tensiden kompatibel, verfügen aber nur über begrenzte pflegende Eigenschaften.

Es besteht daher weiterhin ein Bedarf nach Wirkstoffen bzw. Wirkstoffkombinationen mit guten pflegenden Eigenschaften und guter biologischer Abbaubarkeit, bei denen unerwünschte Kumulationen auf dem Haar ausgeschlossen sind.

Es wurde nun überraschenderweise gefunden, daß eine Kombination aus bestimmten Carbonsäuren oder deren Salzen und wasserunlöslichen Ölen, Fetten oder Wachsen diese Anforderungen in hervorragender Weise erfüllt.

Ein erster Gegenstand der Erfindung ist somit ein Haarbehandlüngsmittel enthaltend

(A) mindestens einer Carbonsäure bzw, deren Salzen gemäß der allgemeinen Formel (I)

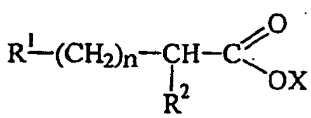
(I)

(B) mindestens einem wasserunlöslichen Öl, Wachs oder Fett sowie als weitere Bestandteile (C) mindestens ein nichtionisches Tensid sowie (D) mindestens ein weiteres anionisches Tensid, mit der Maßgabe, daß die Verbindung der Formel (I) ausgewäht ist aus den Derivaten der Asparaginsäure, der Glutaminsäure sowie der Alkyl- und Alkenylbernsteinsäure.

Die deutsche Offenlegungsschrift DE-A1-44 30 522 beschreibt Mittel zum Färben und Tönen von menschlichen Haaren mit $C_8$- bis $C_{18}$-Acylaminocarbonsäuren. Zwar werden im Rahmen einer Aufzählung üblicher optionaler Komponenten auch Fettalkohole genannt; der Schrift ist aber keinerlei Hinweis auf einen synergistischen Effekt dieser beiden Komponenten bezüglich der Kämmbarkeit der Haare zu entnehmen.

Die EP-A1-830 856 offenbart Haarpflegemittel, die unter anderem N-Acylaminocarbonsäuren enthalten.

Die EP-A1-648 833 offenbart Reinigungsmittel, die N-Acylsubstituierte Derivate von speziellen Aminosäuren enthalten.

Der erste Bestandteil (A) sind Carbonsäuren oder deren Salze gemäß Formel (I), in der n für eine ganze Zahl von 0 bis 9 steht. Der Substituent $R^1$ steht für Wasserstoff, für eine verzweigte oder unverzweigte $C_1$- bis $C_{19}$-Alkylgruppe, beispielsweise für eine der Gruppen Methyl, Ethyl, Propyl, Isopropyl und Butyl, oder für eine verzweigte oder unverzweigte, einfach oder mehrfach ungesättigte $C_1$-bis $C_{19}$-Alkylgruppe, beispielsweise für eine Ethylen- oder Propylengruppe. Weiterhin kann der Substituent $R^1$ für Gruppen der Formel (II) stehen

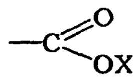 (II)

.

Der Substituent $R^2$ in Formel (I) steht für eine Gruppe der Formel (III)

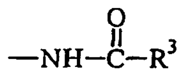 (III)

oder, wenn $R^1$ eine Gruppe der Formel (II) ist, auch für eine $C_4$- bis $C_{22}$-Alkylgruppe oder eine Gruppe der Formel (IV)

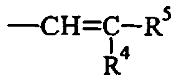 (IV)

wobei der Substituent $R^3$ steht für eine lineare oder verzweigte $C_1$- bis $C_{21}$-Alkylgruppe, beispielsweise für eine Undecyl-, Pentadecyl-, Tridecyl-, oder Heptadecylgruppe oder eine lineare oder verzweigte, einfach oder mehrfach ungesättigte $C_1$- bis $C_{21}$-Alkylgruppe, bevorzugt für eine Undecenyl- oder Heptadecenylgruppe und
die Substituenten $R^4$ bis $R^5$ unabhängig voneinander stehen für Wasserstoff, eine lineare oder verzweigte $C_1$- bis $C_{21}$-Alkylgruppe, beispielsweise für eine Cetyl-, Myristyl- oder Stearylgruppe oder eine lineare oder verzweigte, einfach oder mehrfach ungesättigte $C_1$-bis $C_{21}$-Alkylgruppe, mit der Maßgabe, daß mindestens einer der Reste $R^4$ oder $R^5$ für Wasserstoff steht.

Bei den eingesetzten Verbindungen mit Alkylgruppen kann es sich jeweils um einheitliche Substanzen handeln. Es ist jedoch gegebenenfalls bevorzugt, bei der Herstellung dieser Stoffe von pflanzlichen oder tierischen Rohstoffen auszugehen, so daß man Substanzgemische mit unterschiedlichen, vom jeweiligen Rohstoff abhängigen Alkylkettenlängen erhält. Beispielsweise kann es sich um Mischungen handeln, wie sie aus dem Kokosöl, dem Sojaöl oder aus Talg gewonnen werden.

Die Verbindungen der Formel (I) können erfindungsgemäß in Form ihrer freien Säuren (X steht für Wasserstoff) oder in Form ihrer Salze verwendet werden. In diesem Fall steht X für ein Äquivalent eines Alkali-, Erdalkali- oder Aluminiumkations sowie eines Ammonium-, Hydroxyethylammonium- oder ein Tri(hydroxyethyl)-ammoniumions beziehungsweise der protonierte Form einer alkalisch reagierenden Aminosäure. Beispiele für derartige Aminosäuren sind Arginin, Lysin, Omithin und Histidin. Erfindungsgemäß können die Salze der Verbindung (I) bevorzugt sein.

Verbindungen der Formel (I) sind beispielsweise die Derivate der Asparaginsäure und der Glutaminsäure, sowie die Alkyl- und Alkenylbemsteinsäuren. Bevorzugt sind die $C_{15-20}$-Isoalkenylbernsteinsäure, die $C_{16-18}$-Alkenylbernsteinsäure, sowie die $C_{10}$-, $C_{12}$- und $C_{16}$-Alkenylbemsteinsäure. Weiterhin bevorzugt sind die $C_{16}$- und $C_{18}$-Alkylbernsteinsäure. Ganz besonders bevorzugte Verbindungen der Formel (I) sind die Derivate der Asparaginsäure.

Im Rahmen des ersten Gegenstandes sind Verbindungen des Formel (I) umfaßt, die ausgewählt sind aus den Derivaten der Asparaginsäure und der Glutaminsäure sowie die Alkyl- und Alkenylbernsteinsäuren.

Die zweite Komponente der erfindungsgemäßen Wirkstoffkombination ist ein wasserunlösliches Fett, Wachs oder Öl. Als wasserunlöslich im Sinne der Anmeldung sind solche Stoffe zu verstehen, deren Löslichkeit in Wasser bei 20 °C 1 g/l oder weniger beträgt. Stoffe mit einer Löslichkeit von 0,1 g/l und weniger sind bevorzugt.

Als wasserunlösliche Fette und Öle kommen beispielsweise natürliche Fette und Öle, Mono-, Di- und Triglyceride von gesättigten und ungesättigten Fettsäuren, Ethylenglykolester von Fettsäuren, Fettsäuren, niedrig ethoxylierte Fettsäuren, Fettalkohole, Guerbetalkohole, niedrig ethoxylierte Fettalkohole, Cholesterin und niedrig ethoxylierte Cholesterine, natürliche Wachse wie Bienenwachs, Camaubawachs, Jojobaöl oder Silikonwachse, aber auch synthetische Produkte wie beispielsweise Polydialkyl- oder Polyalkylphenylsiloxane in Betracht. Unter niedrig ethoxylierten Verbindungen sind erfindungsgemäß solche mit einem Ethoxylierungsgrad kleiner als 5 zu verstehen.

Weiterhin sind synthetische Polyolefine erfindungsgemäß bevorzugte wasserunlösliche Fette und Öle.

Als besonders wirksam haben sich Fettsäuren, niedrig ethoxylierte Fettsäuren, Fettalkohole und niedrig ethoxylierte Fettalkohole erwiesen. Als Komponente (B) können auch entsprechende Mischungen verwendet werden.

Beispiele für Komponenten (B) sind Laurylalkohol, Myristylalkohol, Cetylalkohol, Stearylalkohol, Mischungen von Cetyl/Stearylalkohol, Talgfettalkohol, Kokosfettalkohol, Palmitinsäure, Stearinsäure, Jojobaöl, Paraffinöl und Polydimethylsiloxane.

Langkettige Fettalkohole wie Cetylalkohol und Stearylalkohol, deren Mischungen sowie natürliche Fettalkoholmischungen mit einem hohen Gehalt an diesen Komponenten sind bevorzugte Komponenten (B). In einer Ausführungsform enthalten die erfindungsgemäßen Mittel einen $C_8$- bis $C_{22}$-Fettalkohol als Komponente (B).

Nichtionogene Tenside (Komponente (C)) sind beispielsweise

- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- $C_{12}$-$C_{22}$-Fettsäuremono- und -diester von Anlagerungsprodukten von 1 bis 30 Mol Ethylenoxid an Glycerin,
- $C_8$-$C_{22}$-Alkylmono- und -oligoglycoside und deren ethoxylierte Analoga sowie
- Anlagerungsprodukte von 5 bis 60 Mol Ethylenoxid an Rizinusöl und gehärtetes Rizinusöl,
- Aminoxide.

Alkylpolyglykoside gemäß der Formel (V)

$$R^6\text{-O-}(Z)_x \qquad (V),$$

in der $R^6$ steht für einen Alkylrest mit 8 bis 22 Kohlenstoffatomen, Z für einen Mono- oder Oligosaccharid und x für eine Zahl von 1,1 bis 5, sind besonders bevorzugte nichtionogene Tenside. Diese Verbindungen sind durch die folgenden Parameter gekennzeichnet.

Der Alkylrest $R^6$ enthält 8 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können lediglich einen bestimmten Alkylrest $R^6$ enthalten. Üblicherweise werden diese Verbindungen aber ausgehend von natürlichen Fetten und Ölen oder Mineralölen hergestellt. In diesem Fall liegen als Alkylreste $R^6$ Mischungen entsprechend den Ausgangsverbindungen bzw. entsprechend der jeweiligen Aufarbeitung dieser Verbindungen vor.

Besonders bevorzugt sind solche Alkylpolyglykoside, bei denen $R^6$

- im wesentlichen aus $C_8$- und $C_{10}$-Alkylgruppen,
- im wesentlichen aus $C_{12}$- und $C_{14}$-Alkylgruppen,
- im wesentlichen aus $C_8$- bis $C_{16}$-Alkylgruppen oder
- im wesentlichen aus $C_{12}$- bis $C_{16}$-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylglykoside mit x-Werten von 1,3 bis 2 sind bevorzugt. Ganz besonders bevorzugt sind Alkylglykoside, bei denen x 1,4 bis 1,6 beträgt.

Erfindungsgemäß weiterhin bevorzugt sind Aminoxide der allgemeine Formel (VI)

$$R^7R^8R^9N \rightarrow O \qquad (VI),$$

wobei $R^7$ für eine verzweigte oder unverzweigte $C_8$- bis $C_{18}$-Alkylkette steht und $R^8$ und $R^9$ unabhängig voneinander stehen für eine $C_1$- bis $C_3$-Alkylgruppe oder eine $C_1$- bis $C_3$-Hydroxyalkylgruppe.

Ein erfindungsgemäßes Aminoxid ist ferner das von der Firma Tego® Cosmetics vertriebene Aminoxid WS 35, wobei $R^8$ und $R^9$ für Methylgruppen stehen und $R^7$ ein Kokosacylamidopropylrest ist.

Ganz besonders bevorzugte nichtionische Tenside sind die Alkylpolyglycoside, die ethoxylierten Fettalkohole sowie die Aminoxide.

Als anionische Tenside (Komponente (D)) eignen sich in erfindungsgemäßen Zubereitungen alle für die Verwendung am menschlichen Körper geeigneten anionischen oberflächenaktiven Stoffe, die in einem schwach sauren Medium löslich sind. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Giykoi- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,

- lineare Fettsäuren mit 10 bis 22 C-Atomen (Seifen),
- Ethercarbonsäuren der Formel $R\text{-}O\text{-}(CH_2\text{-}CH_2O)_x\text{-}CH_2\text{-}COOH$, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobemsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuren und deren Methylester von Fettsäuren mit 12 bis 18 C-Atomen,
- Alkylsulfate und Alkylpolyglykolethersulfate der Formel $R\text{-}O(CH_2\text{-}CH_2O)_x\text{-}SO_3H$, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Eine bevorzugte Gruppe der anionischen Tenside bilden die Acyllactylate der allgemeinen Formel (VII)

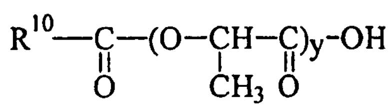

(VII)

,

in der $R^{10}$ für eine lineare oder verzweigte, gesättigte oder ungesättigte Alkyl- oder Alkenylgruppe mit 6 bis 22 Kohlenstoffatomen steht und y für eine Zahl von 1 bis 5, sowie deren physiologisch verträglichen Salze, die sich als besonders wirksam im Sinne der Erfindung erwiesen haben.

Acyllactylate, bei denen $R^{10}$ für eine, insbesondere gesättigte, lineare oder methylverzweigte Alkylgruppe mit 12 bis 18 Kohlenstoffatomen steht und y für eine Zahl von 1 bis 3, sind besonders bevorzugt.

Solche Acyllactylate sind im Handel unter dem Warenzeichen Pationic® erhältlich.

Ein Natrium-isostearoyllactylat, das unter der Bezeichnung Pationic® ISL vertrieben wird, ist ein besonders bevorzugtes Acyllactylat.

Ebenfalls bevorzugte anionische Tenside sind Kondensationsprodukte von organischen Säuren, insbesondere Fettsäuren oder Fettsäuregemischen, an Proteinhydrolysate, insbesondere an Pflanzenproteinhydrolysate. Bevorzugte Säuren sind Ölsäure, Myristinsäure, Laurinsäure, Undecylensäure, Kokosfettsäure und Abietinsäure. Die Kondensationsprodukte können auch in Form von Salzen, insbesondere Natrium-, Kalium- und Triethanolaminsalzen vorliegen.

Erfindungsgemäße Proteinhydrolysate sind beispielsweise Kollagenhydrolysate (CTFA-Bezeichnungen: Hydrolyzed Animal Protein, Hydrolyzed Animal Collagen und Soluble Animal Collagen). Hydrolysate auf Basis Rinder-Kollagen können hierbei bevorzugt sein. Weiterhin erfindungsgemäße Proteinhydrolysate sind Elastin-Hydrolysate (CTFA-Bezeichnung: Hydrolyzed Animal Elastin), Keratin-Hydrolysate (CTFA-Bezeichnung: Hydrolyzed Animal Keratin) sowie die Hydrolysate von Weizenprotein, Milchprotein, Eiweißprotein, Seidenprotein, Mandelprotein, Sojaeiweiß und Proteine aus Tierhäuten. Beispiele für erfindungsgemäße Markenprodukte sind Lamepon®LPO, Lamepon®4 SK, Lamepon®UD, Lamepon®460, Lamepon®PA TR, Lamepon®ST 40, Lamepon®S, Lamepon®PO-TR, Lamepon®PA-K, Lamepon®S-MV und Lamepon®S-TR (Grünau); Lexein®A 200, Lexein®A 240, Lexein®S 620 und Lexein®A 520 (Inolex) sowie Crolastin®AS und Crotein®CCT (Croda).

Weiterhin bevorzugte anionische Tenside sind die Alkylamidopolyoxyethylencarbonsäuren der Formel (VIII)

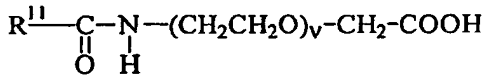

(VIII)

wobei $R^{11}$ für eine verzweigte oder unverzweigte $C_7$- bis $C_{17}$-Alkylgruppe und v für eine Zahl von 1 bis 10 steht. Ein Beispiel für ein Tensid der Formel (VIII) ist das unter dem Handelsnamen Akypo® Foam 30 vertriebene Natriumlaurylamidethercarboxylat (INCI-Bezeichnung: Sodium PEG-4-lauramide carboxylate)

Besonders bevorzugt sind als anionische Tenside bei diesem Gegenstand der Erfindung Alkylethercarbonsäuren, Alkylamidopolyoxyethylencarbonsäuren, Eiweiß-Fettsäure-Kondensationsprodukte und Acyllactylate. Weiterhin sind Salze von gesättigten und insbesondere ungesättigten $C_8$-$C_{22}$-Carbonsäuren, wie Ölsäure, Stearinsäure, Isostearinsäure und Palmitinsäure besonders bevorzugte anionische Tenside.

Als besonders vorteilhaft hat es sich erwiesen, wenn Haarbehandlungsmittel neben dem Wirkstoffkomplex, gebildet aus den Komponenten A und B, weitere Komponenten, wie z.B. ein aminogruppenmodifiziertes Silikonöl, ein Alkylamidoamin oder die Kombination von Stärke mit einem anionischen Tensid enthält.

Ein zweiter Gegenstand der Erfindung sind daher Haarbehandlungsmittel, die neben den oben beschriebenen Komponenten (A) und (B) als weiteren Bestandteil mindestens ein aminogruppenhaltiges Silikonöl, wie beispielsweise die im Handel erhältlichen Produkte Q2-7224 (Hersteller: Dow Coming; ein stabilisiertes Trimethylsilylamodimethicon), Dow Corning 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80) enthalten.

Besonders bevorzugt sind hydroxyl-amino-modifizierte Silikone.

Die aminogruppenhaltigen Silikonöle sind in Mengen von 0,1 bis 10 Gew.-%, bevorzugt von 0,5 bis 5 Gew.-% in den erfindungsgemäßen Mitteln enthalten.

Ein dritter Gegenstand der Erfindung sind Haarbehandlungsmittel, die neben den oben beschriebenen Komponenten (A) und (B) als weiteren Bestandteil mindestens ein Alkylamidoamin enthalten.

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Die eingesetzten Mengen können im Bereich von 0,1-20 Gew.-% variieren.

Ein vierter Gegenstand der Erfindung sind schließlich Haarbehandlungsmittel, die neben den oben beschriebenen Komponenten (A) und (B) als weitere zwingende Bestandteile wasserlösliche Stärke in Mengen von 0,1-10 Gew.-% und mindestens ein anionisches Tensid in Konzentrationen von 0,1-20 Gew.-% enthalten.

Im Rahmen dieses Gegenstandes der Erfindung können prinzipiell alle oben genannten anionischen Tenside zur Anwendung kommen. Bevorzugte anionische Tenside sind Alkylsulfate, Eiweiß-Fettsäure-Kondensationsprodukte, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten $C_8$-$C_{22}$-Carbonsäuren, wie Ölsäure, Stearinsäure, Isostearinsäure und Palmitinsäure.

Die Art des erfindungsgemäß verwendeten Haarbehandlungsmittels unterliegt keinen Beschränkungen. Die erfindungsgemäßen Mittel können sowohl auf dem Haar verbleiben, als auch nach einer Einwirkzeit von wenigen Sekunden bis Minuten wieder ausgespült werden. Beispiele für erfindungsgemäß verwendete Mittel sind Shampoos, Spülungen, Kuren, Konditioniermittel, Tönungsmittel, Färbemittel, Dauerwellmittel, Fixiermittel, Haarsprays und Fönwellen.

In einer bevorzugten Ausführungsform können die erfindungsgemäßen Mittel mindestens ein Oxidationsfarbstoffvorprodukt und/oder mindestens einen direktziehenden Farbstoff enthalten und zum Färben oder Tönen der menschlichen Haare dienen.

Als Vorprodukte für Oxidationsfarbstoffe enthalten Haarfärbemittel sogenannte Entwickler- und Kupplerkomponenten. Die Entwicklerkomponenten bilden unter dem Einfluß von Oxidationsmitteln oder Luftsauerstoff, gegebenenfalls mit Hilfe spezieller Enzyme oder Metallionen als Katalysator, untereinander oder unter Kupplung mit einer oder mehreren Kupplerkomponenten die eigentlichen Farbstoffe aus.

Erfindungsgemäß bevorzugte Entwicklerkomponenten sind p-Phenylendiamin, p-Toluylendiamin, p-Aminophenol, o-Aminophenol, 1-(2'-Hydroxyethyl)-2,5-diaminobenzol, N,N-Bis-(2-hydroxy-ethyl)-p-phenylendiamin, 2-(2,5-Diaminophenoxy)-ethanol, 1-Phenyl-3-carboxyamido-4-amino-pyrazolon-5, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxyethylaminomethyl-4-amino-phenol, 4,4'-Diaminodiphenylamin, 4-Amino-3-fluorphenol, 2-Aminomethyl-4-amino-phenol, 2-Hydroxymethyl-4-aminophenol,

Bis-(2-hydroxy-5-aminophenyl)-methan, 1,4-Bis-(4-aminophenyl)-diazacycloheptan, 1,3-Bis(N(2-hydroxyethyl)-N (4-aminophenylamino))-2-propanol, 4-Amino-2-(2-hydroxyethoxy)-phenol sowie 4,5-Diaminopyrazol-Derivate nach EP 0 740 741 bzw. WO 94/08970 wie z.B. 4,5-Diamino-1-(2'-hydroxyethyl)-pyrazol.

Ganz besonders bevorzugte Entwicklerkomponenten sind p-Phenylendiamin, p-Toluylendiamin, p-Aminophenol, 1-(2'-Hydroxyethyl)-2,5-diaminobenzol, 4-Amino-3-methylphenol, 2,4,5,6-Tetraaminopyrimidin, 2-Hydroxy-4,5,6-triaminopyrimidin und 4-Hydroxy-2,5,6-triaminopyrimidin. 2-Aminomethyl-4-aminophenol ist ebenfalls eine ganz besonders bevorzugte Entwicklerkomponente.

Erfindungsgemäß bevorzugte Kupplerkomponenten sind 1-Naphthol, Pyrogallol, 1,5-, 2,7-und 1,7-Dihydroxynaphthalin, o-Aminophenol, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 4-Chlorresorcin, 2-Chlor-6-methyl-3-aminophenol, 2-Methylresorcin, 5-Methylresorcin, 2,5-Dimethylresorcin, 2,6-Dihydroxypyridin, 2,6-Diaminopyridin, 2-Amino-3-hydroxypyridin, 2,6-Dihydroxy-3,4-diaminopyridin, 3-Amino-2-methylamino-6-methoxypyridin, 4-Amino-2-hydroxytoluol, 2,6-Bis-(2-hydroxyethylamino)-toluol, 2,4-Diaminophenoxyethanol, 1-Methoxy-2-amino-4-(2-hydroxyethylamino)-benzol, 2-Methyl-4-chlor-5-amino-phenol, 6-Methyl-1,2,3,4-tetrahydro-chinoxalin, 3,4-Methylendioxyphenol, 3,4-Methylendioxyanilin, 2,6-Dimethyl-3-amino-phenol, 3-Amino-6-methoxy-2-methylaminophenol, 2-Hydroxy-4-aminophenoxyethanol, 2-Methyl-5-(2-hydroxyethylamino)-phenol und 2,6-Dihydroxy-3,4-dimethylpyridin.

Ganz besonders bevorzugte Kupplerkomponenten sind 1-Naphthol, 1,5-, 2,7- und 1,7-Dihydroxynaphthalin, 5-Amino-2-methylphenol, Resorcin, 4-Chlorresorcin, 2-Chlor-6-methyl-3-aminophenol, 2-Methylresorcin, 5-Methylresorcin, 2,5-Dimethylresorcin, 2,6-Dihydroxy-3,4-diaminopyridin und 6-Methyl-1,2,3,4-tetrahydro-chinoxalin. Weiterhin sind 2-Amino-3-hydroxypyridin und 3-Amino-2-methylamino-6-methoxypyridin ganz besonders bevorzugte Kupplerkomponenten.

Die erfindungsgemäßen Haarfärbemittel enthalten sowohl die Entwicklerkomponenten als auch die Kupplerkomponenten bevorzugt in einer Menge von 0,005 bis 20 Gew.-%, jeweils bezogen auf das Färbemittel ohne die Oxidationsmittelzubereitung. Dabei werden Entwicklerkomponenten und Kupplerkomponenten im allgemeinen in etwa molaren Mengen zueinander eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten in einem MolVerhältnis von 1 : 0,5 bis 1 : 2 enthalten sein können.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen Persulfate, Chlorite und insbesondere Wasserstoffperoxid oder dessen Anlagerungsprodukte an Harnstoff, Melamin sowie Natriumborat in Frage. Weiterhin ist es möglich, die Oxidation mit Hilfe von Enzymen durchzuführen. Dabei können die Enzyme zur Übertragung von Luftsauerstoff auf die Entwicklerkomponente oder zur Verstärkung der Wirkung geringer Mengen vorhandener Oxidationsmittel dienen. Ein Beispiel für ein enzymatisches Verfahren stellt das Vorgehen dar, die Wirkung geringer Mengen (z.B. 1 % und weniger, bezogen auf das gesamte Mittel) Wasserstoffperoxid durch Peroxidasen zu verstärken.

Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Violett-1,4 D, Disperse Black 9, Basic Brown 16 und Basic Brown 17 bekannten Verbindungen sowie 4-Amino-2-nitrodiphenylamin-2'-carbonsäure, 6-Nitro-1,2,3,4-tetrahydrochinoxalin, Hydroxyethyl-2-nitro-toluidin, Pikraminsäure, 2-Amino-6-chloro-4-nitrophenol 4-Ethylamino-3-nitrobenzoesäure und 2-Chloro-6-ethylamino-1-hydroxy-4-nitrobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Weitere in den erfindungsgemäßen Färbemitteln enthaltene Farbstoffkomponenten können auch Indole und Indoline, sowie deren physiologisch verträgliche Salze, sein. Bevorzugte Beispiele sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol. Weiterhin bevorzugt sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 6-Hydroxyindolin, 6-Aminoindolin und 4-Aminoindolin.

In einer weiteren bevorzugten Ausführungsform können die erfindungsgemäßen Haarbehandlungsmittel, gegebenenfalls zusätzlich, zum dauerhaften Verformen von menschlichen Haaren dienen.

Eine dauerhafte Haarverformung wird nach den bekannten Dauerwell-Verfahren in der Weise durchgeführt, daß man das Haar mechanisch verformt und die Verformung z. B. durch Aufwickeln auf Haarwickler oder Papilloten

festlegt. Vor und/oder nach dieser Verformung behandelt man das Haar mit der wäßrigen Zubereitung einer keratinreduzierenden Substanz und spült nach einer Einwirkungszeit mit Wasser oder einer wäßrigen Lösung. In einem zweiten Schritt behandelt man dann das Haar mit der wäßrigen Zubereitung eines Oxidationsmittels. Nach einer Einwirkungszeit wird auch dieses aus dem Haar ausgespült und das Haar von den mechanischen Verformungshilfsmitteln (Wickler, Papilloten) befreit.

Die wäßrige Zubereitung des Keratinreduktionsmittels ist üblicherweise alkalisch eingestellt, damit das Haar quillt und auf diese Weise ein tiefes Eindringen der keratinreduzierenden Substanz in das Haar ermöglicht wird. Die keratinreduzierende Substanz spaltet einen Teil der Disulfid-Bindungen des Keratins zu -SH-Gruppen, so daß es zu einer Lockerung der Peptidvemetzung und infolge der Spannung des Haares durch die mechanische Verformung zu einer Neuorientierung des Keratingefüges kommt. Unter dem Einfluß des Oxidationsmittels werden emeut Disulfid-Bindungen geknüpft, und auf diese Weise wird das Keratingefüge in der vorgegebenen Verformung neu fixiert.

Erfindungsgemäß können die erfindungsgemäßen Wirkstoffkombinationen sowohl in dem keratinreduzierenden (Wellmittel) als auch in dem keratinoxidierenden Mitteln (Fixierlösung) der Dauerwellbehandlung eingearbeitet sein. Besonders bevorzugt ist der Einsatz der erfindungsgemäßen Wirkstoffkombinationen in Fixierlösungen

Als Wellmittel werden heute fast ausschließlich alkalisch eingestellte (pH-Werte von 8,5 bis 9,5) Lösungen von Thioglycolsäure oder deren Salzen oder Estern sowie Thiomilchsäure, Cysteamin, Thioäpfelsäure oder $\alpha$-Mercaptoethansoulfonsäure verwendet.

Zwingender Bestandteil der Fixiermittel sind Oxidationsmittel, z. B. Natriumbromat, Kaliumbromat, Wasserstoffperoxid, und die zur Stabilisierung wäßriger Wasserstoffperoxidzubereitungen üblichen Stabilisatoren. Der pH-Wert solcher wäßriger $H_2O_2$-Zubereitungen, die üblicherweise etwa 0,5 bis 3,0 Gew.-% $H_2O_2$ enthalten, liegt bevorzugt bei 2 bis 4; er wird durch anorganische Säuren, bevorzugt Phosphorsäure, eingestellt. Fixiermittel auf Bromat-Basis enthalten die Bromate üblicherweise in Konzentrationen von 1 bis 10 Gew.-% eingesetzt und der pH-Wert der Lösungen wird auf 4 bis 7 eingestellt. Gleichfalls geeignet sind Fixiermittel auf enzymatischer Basis (Peroxidasen), die keine oder nur geringe Mengen an Oxidationsmitteln, insbesondere $H_2O_2$, enthalten.

Weitere Wirk-, Hilfs- und Zusatzstoffe sind beispielsweise

- kationische Tenside wie quartäre Ammoniumverbindungen, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Cetylpyridiniumchlorid, sowie sogenannte "Esterquats", z.B. Dehyquart®AU-46, Dehyquart®F-30 und Dehyquart®F-75 und Proteinhydrolysate.
- zwitterionische Tenside wie die sogenannten Betaine, beispielsweise das Kokosalkyl-dimethylammonium-glycinat und das Kokosacylaminopropyldimethylammoniumglycinat
- ampholytische Tenside, beispielsweise das N-Kokosalkylaminopropionat, das Kokosacylaminoethylaminopropionat und das $C_{12-18}$-Acylsarcosin.
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- permanent kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert.Butylaminoethylmethacrylat/2-Hydroxypropylmethacrylat-Copolymere,
- anionische Polymere wie beispielsweise Polyacrylsäuren, vemetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,

- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Farbstoffe zum Einfärben der Zubereitungen,
- Treibmittel wie Propan-Butan-Gemische, $N_2O$, Dimethylether, $CO_2$ und Luft sowie
- Antioxidantien.

Die folgenden Beispiele sollen den Gegenstand der Erfindung näher erläutern.

## Ausführungsbeispiele

Alle Mengenangaben in den folgenden Rezepturen sind Gewichtsanteile.

1. Spülung mit langanhaltender Pflegewirkung

| Rohstoff | Rezeptur 6 |
|---|---|
| N-Cocoyl-L-glutaminsäure, Natriumsalz, 25%-ig in Wasser | 8,0 |
| Hydrenol® D | 5,0 |
| Kationische Emulsion DC 929[4] | 3,0 |
| Wasser | ad 100,0 |

[4] Hydroxyl-Amino-modifiziertes Silicon (INCI-Bezeichnung: Aminodimethicone (and) Tallowtrimonium chloride (and) Nonoxynol-10) (Dow Coming)

Die Naßkämmbarkeit der Rezeptur 6 wurde unmittelbar nach dem Ausspülen mit der Note 1 in der oben beschriebenen Weise bewertet. Nach einer Shampoonierung mit einem handelsüblichen Shampoo resultierte die Note 2, nach drei Shampoonierungen die Note 3-4.

2. Haarkur mit Alkylamidoamin

| Rohstoff | Rezeptur 7 |
|---|---|
| N-Cocoyl-L-glutaminsäure, Natriumsalz, 25%-ig in Wasser | 8,0 |
| Kokosfettalkohol | 6,0 |
| Paraffinöl, dünnflüssig | 2,0 |
| Tegoamid® S18[5] | 1,0 |
| Citronensäure | ad pH 4.5 |
| Wasser | ad 100,0 |

[5] N,N-Dimethyl-N'-stearoyl-1,3-diaminopropan (INCI-Bezeichnung: Stearamidopropyl Dimethylamin) (Tego Cosmetics)

Die Einwirkdauer auf dem handtuchtrockenen Haar betrug 20 Minuten. Die Naßkämmbarkeit wurde mit 1

bewertet.

3. Shampoo mit kämmbarkeitsverbessernder Wirkung und verbessertem Frisurenhalt

| Rohstoff | Rezeptur 8 |
|---|---|
| N-Cocoyl-L-asparaginsäure, Kaliumsalz, 25%ig in Wasser | 4,0 |
| Texapon® NSO | 20,0 |
| Plantaren® 700[6] | 2,0 |
| Akyposoft® 45 NV[7] | 3,0 |
| Stenol® 1618[8] | 3,0 |
| Eumulgin® B1[9] | 0,5 |
| Eumulgin® B2[10] | 0,5 |
| Aminoxid WS 35[11] | 2,0 |
| Pationic® ISL[12] | 3,0 |
| Gluadin® W40[13] | 2,0 |
| Konservierungsmittel, Komplexierungsmittel, Parfümöl, Wasser | ad 100,0 |

[6] $C_8$- bis $C_{10}$-Fettalkohol-1.4-glucosid (HENKEL)
[7] $C_{12}$- bis $C_{14}$-Fettalkohol mit 4,5 EO-Einheiten-Essigsäure-Natriumsalz (INCI-Bezeichnung: Sodium Laureth-6 Carboxylate) (KAO)
[8] $C_{16}$- bis $C_{18}$- Fettalkohol (INCI-Bezeichnung: Cetearylakohol) (HENKEL)
[9] Cetylstearyalkohol mit 12 EO-Einheiten (INCI-Bezeichnung: Ceteareth 12) (HENKEL)
[10] Cetylstearylalkohol mit 20 EO-Einheiten (INCI-Bezeichnung: Ceteareth-20) (HENKEL)
[11] N,N-Dimethyl-N-($C_{8-18}$-kokosacylamidopropyl)amin-N-oxid (INCIBezeichnung: Cocoamidopropylamine Oxide) (Tego Cosmetics)
[12] Natriumsalz des Isostearyllactats (INCI-Bezeichnung: Sodium Isostearoyl Lactylate) (PATCO Products)
[13] Weizenproteinhydrolysat (INCI-Bezeichnung: Aqua (Water), Hydrolized wheat protein, sodium benzoate, phenoxycthanol, methylparaben, propylparaben) (HENKEL)

Das Shampoo weist sehr gute Pflegeeigenschaften (Griff, Naßkämmbarkeit) und einen verbesserten Frisurenhalt auf.

4. Haartönungsmittel

| Rohstoff | Rezeptur 9 |
|---|---|
| N-Cocoyl-L-glutaminsäure, Natriumsalz, 25%ig in Wasser | 5,0 |
| Stenol® 1618 | 4,0 |
| Kationische Emulsion DC929 | 2,0 |
| Laurinsäure | 2,0 |
| Texapon® NSO | 10,0 |
| Akyposoft® 45NV | 5,0 |
| Violett-1,4 D[14] | 1,0 |
| HC Blue 2[15] | 1,0 |
| Arianor® Stahlblau[16] | 0,2 |

[14] N,N'-Bis-(2-hydroxyethyl)-2-nitro-1,4-diaminobenzol
[15] N,N',N'-Tris-(2-hydroxyethyl)-2-nitro-1,4-diaminobenzol
[16] Farbstoff C.I. 56059

(fortgesetzt)

| Rohstoff | Rezeptur 9 |
|---|---|
| $NaHCO_3$ | 0,5 |
| Ammoniaklösung, 25%ig | ad pH 9,5 |
| Parfümol, Komplexierungsmittel, Wasser | ad 100,00 |

1g des Tönungsmittels wurde auf eine ca. 0,5g schwere Haarsträhne (Kerling, naturweiß) gegeben. Nach 20min wurde abgespült und getrocknet. Die Haarsträhne zeigte eine intensive blauviolette Nuancierung.
Die Prüfung der Naßkämmbarkeit nach 20min Einwirkdauer ergab eine Note von 2-3.

5. Haarfärbemittel

| Rohstoff | Rezeptur 10 |
|---|---|
| Hydrenol® D | 8,0 |
| Lorol® techn.[17] | 2,0 |
| Eumulgin® B1 | 1,0 |
| Eumulgin® B2 | 1,0 |
| N-Cocoyl-L-asparaginsäure, Kaliumsalz, 25%ig in Wasser | 12,0 |
| Akyposoft® 45NV | 4,0 |
| Isostearinsäure | 2,0 |
| Kationische Emulsion DC929 | 4,0 |
| Meadowfoam seed oil | 2,0 |
| Ammoniumsulfat | 0,5 |
| EDTA | 0,1 |
| 1-Methyl-2,5-diaminobenzol-sulfat | 0,15 |
| 4-Aminophenol-hydrochlorid | 0,26 |
| Resorcin | 0,11 |
| 5-Amino-2-methylphenol | 0,1 |
| Ascorbinsäure | 0,5 |
| Ammoniaklösung, 25%ig | ad pH 9,8 |
| Parfümöl, Wasser | ad 100,0 |

[17] $C_{12}$- bis $C_{18}$-Fettalkohol (INCI-Bezeichnung: Coconut alcohol) (HENKEL)

Ausgefärbt wurde auf Strähnen (Typ Kerling, naturweiß) nach Mischen mit einer 6%igen kommerziellen Entwicklerdispersion (Poly Color Intensiv Color Creme) im Verhältnis 1:1 (2g Färbemischung auf 0,5g Haar). Nach einer Einwirkzeit von 30min wurde die Strähne mit Wasser gespült und getrocknet. Es resultierte ein Kupferton.
Die Prüfung der Naßkämmbarkeit erfolgte separat an speziell vorbehandelten Strähnen nach dem oben beschriebenen Verfahren. Die Beurteilung erfolgte nach einer 30minütigen Einwirkzeit der Färbemischung (4g) auf 1g Strähne und sie ergab die Note 3.

6. Wellotion

| Rohstoff | Rezeptur 11 | Rezeptur 12 |
|---|---|---|
| Stenol® 1618 | 0,8 | 0,8 |
| Eumulgin® B1 | 0,2 | 0,2 |
| Eumulgin® B2 | 0,2 | 0,2 |
| N-Cocoyl-L-asparaginsäure, Kaliumsalz, 25%ig in Wasser | 1,5 | -- |
| Lamepon® S[18] | 1,5 | 3,0 |
| Siliconöl DC345[19] | -- | 1,0 |
| Kationische Emulsion DC 929 | 1,0 | -- |
| EDTA | 0,3 | 0,3 |
| Parfümöl | 0,2 | 0,2 |
| Harnstoff | 3,0 | 3,0 |
| Thioglycolsäure | 8,0 | 8,0 |
| Ammoniaklösung, 25%ig | ad pH 8,5 | ad pH 8,5 |
| Wasser | ad 100,0 | ad 100,0 |

[18] Eiweiß-Fettsäurekondensat (INCI-Bezeichnung: Potassium Cocoyl Hydrolysed Collagen) (HENKEL)
[19] Dimethylcyclosiloxan-Pentamer (INCI-Bezeichnung: Cyclomethicone (Dow Corning)

Nach der Fixierung mit einer kommerziellen Fixierlösung (Poly Lock Normal) wies das mit der erfindungsgemäßen Wellcreme (Rezeptur 11) behandelte Haar zweier Modelle noch einen spürbaren Pflegeeffekt (Griff, Naßkämmbarkeit) auf. Bei der Vergleichsrezeptur 12 (nicht erfindungsgemäß) konnte dieser Pflegeeffekt nicht festgestellt werden.

7. Fixierung

| Rohstoff | Rezeptur 13 | Rezeptur 14 |
|---|---|---|
| Stenol® 1618 | 2,0 | 2,0 |
| N-Cocoyl-L-asparaginsäure, Kaliumsalz, 25%ig in Wasser | 10,0 | -- |
| Texapon® NSO | -- | 10,0 |
| Kationische Emulsion DC 929 | 2,0 | 2,0 |
| Wassertoffperoxid, 50%ig | 5,0 | 5,0 |
| Phosphorsäure | ad pH 5,0 | ad pH 5,0 |
| Komplexierungsmittel, Wasser | ad 100 | ad 100 |

Die Prüfung nach Anwendung einer kommerziellen Wellösung (Poly Lock, Normalvariante) an nicht vorgeschädigtem Haar zweier Modelle im Haarstudio ergab mit der erfindungsgemäßen Fixierlösung 13 einen spürbaren Pflegeeffekt (Griff, Naßkämmbarkeit). Ersetzt man in der Fixierlösung das Asparaginsäurederivat durch ein Alkylethersulfat (Rezeptur 14, nicht erfindungsgemäß), so geht der Pflegeeffekt weitestgehend verloren.

## Patentansprüche

**1.** Haarbehandlungsmittel zur Verbesserung der Kämmbarkeit, enthaltend

(A) mindestens eine Verbindung gemäß Formel (I),

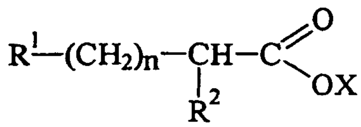

(I)

wobei n eine ganze Zahl von 0 bis 9 ist,
der Substituent $R^1$ ausgewählt ist aus

- Wasserstoff
- einer verzweigten oder unverzweigten $C_1$- bis $C_{19}$-Alkylgruppe
- einer verzweigten oder unverzweigten, einfach oder mehrfach ungesättigten $C_1$- bis $C_{19}$-Alkylgruppe oder
- einer Gruppe der Formel (II)

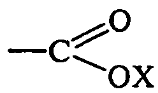

(II)

,

der Substituent $R^2$ für

- eine Gruppe der Formel (III)

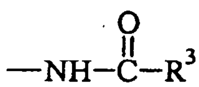

(III)

oder
-wenn $R^1$ eine Gruppe der Formel (II) ist, auch für eine $C_4$- bis $C_{22}$-Alkylgruppe oder eine Gruppe der Formel (IV)

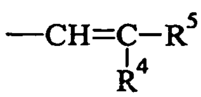

(IV)

steht, wobei der Substituent $R^3$ steht für eine lineare oder verzweigte $C_1$-bis $C_{21}$-Alkylgruppe oder eine lineare oder verzweigte, einfach oder mehrfach ungesättigte $C_1$- bis $C_{21}$-Alkylgruppe,
-die Substituenten $R^4$ bis $R^5$ unabhängig voneinander stehen für Wasserstoff, eine lineare oder verzweigte $C_1$- bis $C_{21}$-Alkylgruppe oder eine lineare oder verzweigte, einfach oder mehrfach ungesättigte $C_1$- bis $C_{21}$-Alkylgruppe, mit der Maßgabe, daß mindestens einer der Reste $R^4$ oder $R^5$ für Wasserstoff steht, und X für

- Wasserstoff,
- ein Äquivalent eines Alkali-, Erdalkali- oder Aluminiumkations oder
- ein Ammonium-, Hydroxyethylammonium- oder ein Tri(hydroxyethyl)-ammoniumion oder
- eine protonierte Form einer alkalisch reagierenden Aminosäure

steht,
(B) mindestens ein wasserunlösliches Öl, Fett oder Wachs,
(C) mindestens ein nichtionisches Tensid sowie
(D) mindestens ein weiteres anionisches Tensid,

mit der Maßgabe, dass die Verbindung der Formel (I) ausgewählt ist aus den Derivaten der Asparaginsäure, der

Glutaminsäure sowie der Alkyl- und Alkenylbemsteinsäure.

**2.** Haarbehandlungsmittel nach Anspruch 1, **dadurch gekennzeichnet, daß** es als nichtionisches Tensid ein Alkylpolyglycosid, ein ethoxylierten Fettalkohol und/oder ein Aminoxid enthält.

**3.** Haarbehandlungsmittel nach einem der Ansprüche 1 oder 2, **dadurch gekennzeichnet, daß** es als anionisches Tensid ein Derivat einer Alkylethercarbonsäure, einer Alkylamidopolyoxyethercarbonsäure, eines Eiweiß-Fettsäure-Kondensationsprodukt, ein Salze einer gesättigten und insbesondere ungesättigten $C_8$-$C_{22}$-Carbonsäure und/oder ein Acyllactylat enthält.

**4.** Haarbehandlungsmittel zur Verbesserung der Kämmbarkeit, enthaltend

(A) mindestens eine Verbindung gemäß Formel (I),
(B) mindestens ein wasserunlösliches Öl, Fett oder Wachs sowie
(C) mindestens ein aminogruppenmodifiziertes Silikonöl.

**5.** Haarbehandlungsmittel zur Verbesserung der Kämmbarkeit, enthaltend

(A) mindestens eine Verbindung gemäß Formel (I),
(B) mindestens ein wasserunlösliches Öl, Fett oder Wachs sowie
(C) mindestens ein Alkylamidoamin.

**6.** Haarbehandlungsmittel zur Verbesserung der Kämmbarkeit, enthaltend

(A) mindestens eine Verbindung der Formel (I),
(B) mindestens ein wasserunlösliches Öl, Fett oder Wachs
(C) Stärke sowie
(D) mindestens ein weiteres anionisches Tensid.

**7.** Haarbehandlungsmittel nach einem der Ansprüche 2 bis 6, **dadurch gekennzeichnet, daß** es mindestens ein Oxidatonsfarbstoffvorprodukt und/oder mindestens einen direktziehenden Farbstoff enthält.

**8.** Haarbehandlungsmittel nach einem der Ansprüche 2 bis 7, **dadurch gekennzeichnet, daß** es zum dauerhaften Verformen von menschlichen Haaren dient.

**9.** Haarbehandlungsmittel nach einem der Ansprüche 2 bis 8, **dadurch gekennzeichnet, daß** es als Komponente (A) Derivate der Asparaginsäure enthält.

**10.** Haarbehandlungsmittel nach einem der Ansprüche 2 bis 9, **dadurch gekennzeichnet, daß** es als Komponente (B) einen $C_8$- bis $C_{22}$-Fettalkohol enthält.

## Claims

**1.** Hair treatment preparation for improving combability containing

(A) at least one compound corresponding to formula (I):

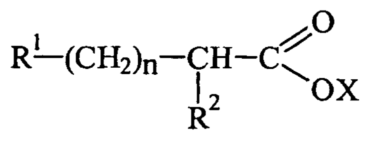

(I)

where n is an integer of 0 to 9,
the substituent $R^1$ is selected from

- hydrogen,
- a branched or unbranched $C_{1-19}$ alkyl group,
- a branched or unbranched, mono- or polyunsaturated $C_{1-19}$ alkyl group or
- a group corresponding to formula (II):

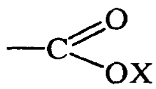

(II)

the substituent $R^2$ is

- a group corresponding to formula (III):
  or
- where $R^1$ is a group of formula (II), also stands for a $C_{4-22}$ alkyl group or a group corresponding to formula (IV):

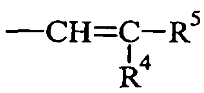

(IV)

the substituent $R^3$ being a linear or branched $C_{1-21}$ alkyl group or a linear or branched, mono- or polyunsaturated $C_{1-21}$ alkyl group, the substituents $R^4$ and $R^5$ independently of one another represent hydrogen, a linear or branched $C_{1-21}$ alkyl group or a linear or branched, mono- or polyunsaturated $C_{1-21}$ alkyl group, with the proviso that at least one of the substituents $R^4$ or $R^5$ is hydrogen, and X is

- hydrogen,
- an equivalent of an alkali metal, alkaline earth metal or aluminium cation or
- an ammonium, hydroxyethylammonium or tri(hydroxyethyl)-ammonium ion or
- a protonated form of an alkaline reacting amino acid,

(B) at least one water-insoluble oil, fat or wax,
(C) at least one nonionic surfactant and
(D) at least one other anionic surfactant,

with the proviso that the compound of formula (I) is selected from derivatives of aspartic acid, glutamic acid and alkyl and alkenyl succinic acid.

**2.** A hair treatment preparation as claimed in claim 1, **characterized in that** it contains an alkyl polyglycoside, an ethoxylated fatty alcohol and/or an amine oxide as the nonionic surfactant.

**3.** A hair treatment preparation as claimed in claim 1 or 2, **characterized in that** it contains a derivative of an alkylether carboxylic acid, an alkylamidopolyoxyether carboxylic acid, a protein fatty acid condensate, a salt of a saturated and, more particularly, unsaturated $C_{8-22}$ carboxylic acid and/or an acyl lactylate.

**4.** A hair treatment preparation for improving combability containing

(A) at least one compound corresponding to formula (I),
(B) at least one water-insoluble oil, fat or wax and
(C) at least one amino-group-modified silicone oil.

**5.** A hair treatment preparation for improving combability containing

(A) at least one compound corresponding to formula (I),
(B) at least one water-insoluble oil, fat or wax and
(C) at least one alkyl amidoamine.

**6.** A hair treatment preparation for improving combability containing

(A) at least one compound corresponding to formula (I),
(B) at least one water-insoluble oil, fat or wax and
(C) starch and
(D) at least one other anionic surfactant.

**7.** A hair treatment preparation as claimed in any of claims 1 to 6, **characterized in that** it contains at least one oxidation dye precursor and/or at least one substantive dye.

**8.** A hair treatment preparation as claimed in any of claims 1 to 7, **characterized in that** it is used for the permanent shaping of human hair.

**9.** A hair treatment preparation as claimed in any of claims 1 to 8, **characterized in that** it contains derivatives of aspartic acid as component (A).

**10.** A hair treatment preparation as claimed in any of claims 1 to 9, **characterized in that** it contains a $C_{8-22}$ fatty alcohol as component (B).

## Revendications

**1.** Agent de traitement de cheveux destiné à améliorer l'aptitude au peignage, contenant

(A) au moins un composé selon la formule (I),

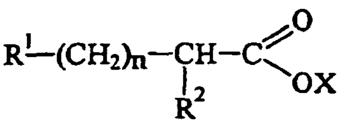

(I)

dans laquelle n représente un nombre entier de 0 à 9,
le substituant $R^1$ est choisi parmi

- l'hydrogène
- un groupe alkyle en $C_1$ à $C_{19}$ ramifié ou non ramifié
- un groupe alkyle en $C_1$ à $C_{19}$ ramifié ou non ramifié, monoinsaturé ou polyinsaturé ou
- un groupe de formule (II)

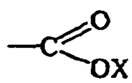

(II)

le substituant $R^2$ représente

- un groupe de formule (III)

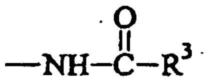

(III)

ou,
- lorsque $R^1$ représente un groupe de formule (II), $R^2$ représente également un groupe alkyle en $C_4$ à $C_{22}$ ou un groupe de formule (IV)

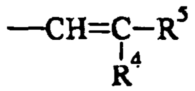 (IV)

où le substituant $R^3$ représente un groupe alkyle en $C_1$ à $C_{21}$ linéaire ou ramifié ou un groupe alkyle en $C_1$ à $C_{21}$ linéaire ou ramifié, monoinsaturé ou polyinsaturé,

- les substituants $R^4$ et $R^5$ représentent indépendamment l'un de l'autre, l'hydrogène, un groupe alkyle en $C_1$ à $C_{21}$ linéaire ou ramifié ou un groupe alkyle en $C_1$ à $C_{21}$ linéaire ou ramifié, monoinsaturé ou polyinsaturé, à condition qu'au moins un des radicaux $R^4$ ou $R^5$ représente l'hydrogène,

et X représente

- l'hydrogène
- un équivalent d'un cation alcalin, alcalino-terreux ou d'aluminium ou
- un ion d'ammonium, d'hydroxyéthylammonium ou de tri(hydroxyéthyl)ammonium ou
- une forme protonée d'un acide aminé à réaction alcaline,

(B) au moins une huile, une graisse ou une cire insoluble dans l'eau,
(C) au moins un agent tensioactif non ionique ainsi que
(D) au moins un autre agent tensioactif anionique,

à condition que le composé de formule (I) soit choisi parmi les dérivés de l'acide aspartique, de l'acide glutamique ainsi que de l'acide alkylsuccinique et de l'acide alcénylsuccinique.

**2.** Agent de traitement des cheveux selon la revendication 1, **caractérisé en ce qu'**il contient un alkylpolyglycoside, un alcool gras éthoxylé et/ou un oxyde d'amine comme agent tensioactif non ionique.

**3.** Agent de traitement des cheveux selon l'une quelconque des revendications 1 ou 2, **caractérisé en ce qu'**il contient un dérivé d'un acide alkyléthercarboxylique, d'un acide alkylamidopolyoxyéthercarboxylique, d'un produit de condensation de protéine et d'acide gras, un sel d'un acide carboxylique en $C_8$ à $C_{22}$ saturé et en particulier insaturé et/ou un lactylate d'acyle comme agent tensioactif anionique.

**4.** Agent de traitement des cheveux destiné à l'amélioration de l'aptitude au peignage, contenant

(A) au moins un composé selon la formule (I),
(B) au moins une huile, une graisse ou une cire insoluble dans l'eau ainsi que
(C) au moins une huile de silicone modifiée par des groupes amino.

**5.** Agent de traitement des cheveux destiné à l'amélioration de l'aptitude au peignage, contenant

(A) au moins un composé selon la formule (I),
(B) au moins une huile, une graisse ou une cire insoluble dans l'eau ainsi que
(C) au moins une alkylamidoamine.

**6.** Agent de traitement des cheveux destiné à l'amélioration de l'aptitude au peignage, contenant

(A) au moins un composé selon la formule (I),
(B) au moins une huile, une graisse ou une cire insoluble dans l'eau,
(C) de l'amidon ainsi que
(D) au moins un autre agent tensioactif anionique.

**7.** Agent de traitement des cheveux selon l'une quelconque des revendications 1 à 6, **caractérisé en ce qu'**il contient au moins un précurseur d'un colorant d'oxydation et/ou au moins un colorant montant directement sur la fibre.

**8.** Agent de traitement des cheveux selon l'une quelconque des revendications 1 à 7, **caractérisé en ce qu'**il sert à la mise en pli durable de cheveux humains.

**9.** Agent de traitement des cheveux selon l'une quelconque des revendications 1 à 8, **caractérisé en ce qu'**il contient des dérivés de l'acide aspartique comme composant (A).

**10.** Agent de traitement des cheveux selon l'une quelconque des revendications 1 à 9, **caractérisé en ce qu'**il contient un alcool gras en $C_8$ à $C_{22}$ comme composant (B).